# EUROPÄISCHE PATENTSCHRIFT

(11) **EP 2 991 594 B1**
(45) Veröffentlichungstag und Bekanntmachung des Hinweises auf die Patenterteilung: **15.12.2021**
(21) Anmeldenummer: 14726536.7
(22) Anmeldetag: 23.04.2014
(51) Int. Cl.: A61F 2/966

(54) **PUSHER-BAUGRUPPE FÜR EIN EINFÜHRSYSTEM FÜR EIN SELBSTEXPANDIERENDES GEFÄSSIMPLANTAT**
PUSHER ASSEMBLY FOR AN INSERTION SYSTEM FOR A SELF-EXPANDING VASCULAR IMPLANT
ENSEMBLE POUSSOIR POUR UN SYSTÈME D'INTRODUCTION POUR UN IMPLANT VASCULAIRE AUTO-EXPANSIBLE

(30) Priorität: 03.05.2013 DE 102013104565
(43) Veröffentlichungstag der Anmeldung: 09.03.2016
(73) Patentinhaber: JOTEC GmbH, 72379 Hechingen (DE)
(72) Erfinder: LESMEISTER, Rainer, 72827 Wannweil (DE)
(74) Vertreter: Witte, Weller & Partner Patentanwälte mbB
(86) Internationale Anmeldenummer: PCT/EP2014/058258
(87) Internationale Veröffentlichungsnummer: WO 2014/177440

(56) Entgegenhaltungen:
- WO-A1-98/04189
- WO-A1-2007/005799
- WO-A1-2011/008538
- US-A1- 2002 045 929

## Beschreibung

Die vorliegende Erfindung betrifft eine Pusher-Baugruppe für ein Einführsystem für ein selbstexpandierendes Gefäßimplantat, wobei die Pusher-Baugruppe einen Katheterschlauch aufweist, welches ein Lumen zur Aufnahme eines Führungsdrahtes aufweist, sowie einen ersten proximalen und einen sich daran anschießenden zweiten distalen Katheterschlauchabschnitt, wobei der distale Katheterschlauchabschnitt zumindest teilweise zur verschiebbaren Aufnahme eines Gefäßimplantats hierauf vorgesehen ist, und wobei der Katheterschlauch ferner proximal angrenzend zu einem auf den Katheterschlauch geladenen Gefäßimplantat eine Pusher-Anordnung zum Freisetzen des Gefäßimplantats aufweist.

Einführsysteme zum Einführen und Freisetzen von Gefäßimplantaten in Gefäße eines Patienten, welche Einführsystem, die eingangs genannte Pusher-Baugruppe aufweisen, sind im Stand der Technik bekannt. Mit solchen Einführsystemen werden Gefäßimplantate, die auch als endovaskuläre Gefäßstents bzw. Stentgrafts bezeichnet werden, beispielsweise zur Behandlung von Aneurysmen oder zur Offenhaltung von Gefäßen allgemein in die zu behandelnden Gefäße implantiert.

Die gegenwärtig verwendeten Gefäßimplantate bestehen dabei überwiegend aus einem hohlzylindrischen Metallrahmen, dessen Mantelfläche von einem Textil- oder Polymermaterial abgedeckt ist, so dass sich ein hohlzylindrischer Körper ergibt, der auch als Stentgraft oder gecoverter Stent bezeichnet wird, wohingegen geflochtene oder lasergeschnittene oder verzwirbelte Metalldrahtgeflechte, die nicht durch ein Textil- oder Polymermaterial umfangsmäßig bedeckt sind, als Stents bzw. nichtgecoverte/ungecoverte Stents bezeichnet werden.

Zur Implantation wird das Gefäßimplantat radial zusammengedrückt, so dass sich seine Querschnittsfläche deutlich verringert. Das Gefäßimplantat wird zur Einbringung in ein Gefäß eines Patienten mit Hilfe eines Einführsystems in das Gefäß eingebracht und freigesetzt. Auf Grund der Federwirkung des Metallrahmens expandiert das Gefäßimplantat nach der Freisetzung wieder in seine ursprüngliche Form und spannt dabei seine Mantelfläche auf, die sich innen im Blutgefäß verklemmt und entweder das Aneurysma dadurch überspannt bzw. das Blutgefäß durch die Federwirkung offenhält. Für die gewünschte Wirkung eines Gefäßimplantats ist es nicht nur erforderlich, dieses so zu positionieren, dass es sich - insbesondere bei einem Aneurysma - in dem entsprechenden Blutgefäß hinreichend fest verspannen kann, auch die radiale Ausrichtung des Gefäßimplantats ist häufig von entscheidender Bedeutung. Dies ist insbesondere dann der Fall, wenn an oder in der Nähe der Stelle, in die das Gefäßimplantat implantiert werden soll, weitere Gefäße abzweigen, deren Versorgung die Einbringung des Gefäßimplantats nicht beeinträchtigt werden darf. Daher ist es insbesondere an diesen Stellen äußerst kritisch, den Stent bei der Implantation nicht in seiner Längsrichtung zu verschieben.

Zur Implantation werden die Gefäßimplantate, wie bereits weiter oben erwähnt, radial zusammengefaltet und dann mit Hilfe eines Führungsdrahts, Führungsdrahtkatheters, aufweichen das Gefäßimplantat in der Regel geladen wird, sowie einem Anschlagrohr bzw. Pusher-Stab und einem proximalen Handhabungsabschnitt sowie mit Hilfe ggf. weiterer zusätzlicher bekannter Merkmale im Gefäß im zu behandelnden Bereich positioniert. Die richtige Lage des Gefäßimplantats kann dabei beispielsweise über Röntgenmarker kontrolliert werden, die am Führungsdraht, Führungsdrahtkatheter, auf dem Mantel des Stents, oder anderen Stellen vorgesehen sind.

Damit die Gefäßimplantate während der Positionierung in einem zusammengefalteten Zustand verbleiben, sind sie in einer Hülle oder einem Schlauch angeordnet, der das Gefäßimplantat radial nach innen komprimiert. Diese sogenannte Rückzugshülle wird nach dem Positionieren des Gefäßimplantats im Gefäß zurückgezogen, wobei zur Fixierung des Stents axial von einem in proximale Richtung nachgeordneten Anschlagrohr, dem sogenannten Pusher, gehalten wird. Der Pusher liegt dabei in Anlage mit dem Gefäßimplantat und hält diesen in seiner axialen Lage, während die auch den Pusher umgebende Rückzugshülle von dem Gefäßimplantat abgezogen wird, der sich dabei expandiert und in dem Gefäß verklemmt.

Zu Beginn des Implantationsschrittes wird also zunächst ein Führungsdraht in das Gefäß eingeführt und zu dem behandelnden Gefäßbereich vorgeschoben. Sobald der zu behandelnde Gefäßbereich erreicht ist, wird der distale Teil der Einführvorrichtung, also derjenige Teil, der von der handhabenden Person weiter entfernt liegt als der proximale, von der handhabenden Person betätigte Teil des Einführsystems, und welcher distale Teil den Führungsdrahtkatheter, das Gefäßimplantat sowie den Pusher umfasst, über den Führungsdraht in das Gefäß und zu dem behandelnden Bereich geführt. Der Führungsdrahtkatheter ist dabei in der Regel mit einer flexiblen Dilatorspitze an seinem distalen Ende versehen, um die Gefäßwege zu erweitern, so dass die Einführvorrichtung und das Gefäßimplantat darin leichter aufgenommen werden können.

Damit das distale Ende der Einführvorrichtung mit einer gewissen Flexibilität ausgestattet ist, ist in der Regel der Führungsdrahtkatheter, auf welchem das Gefäßimplantat während des Einführens in das Gefäß getragen wird, flexibel, so dass sich insbesondere dieser Teil den Gegebenheiten des behandelnden Gefäßes, insbesondere dessen Krümmungen anpassen kann, sowie dem Führungsdraht, über welchen der Führungsdrahtkatheter eingeführt wird, folgen kann.

Andererseits muss der Pusher aufgrund der hohen Kräfte, die beim Rückziehen der Rückzugshülle und zur Freisetzung des Stents ausgeübt werden, im Vergleich zum Führungsdrahtkatheter in der Regel deutlich steifer sein, um der in proximale Richtung ausgeübten Kraft, die beim Zurückziehen der das Gefäßimplantat komprimierenden Rückzugshülle eingesetzt wird, eine hinreichend starke Anschlagkraft entgegenzusetzen. Aus diesen Gründen ist daher der Pusher in der Regel bedeutend steifer als die anderen Komponenten des Einführsystems.

WO2007/005799 A1 offenbart die Präambel von Anspruch 1. Die üblicherweise im Stand der Technik eingesetzten Rückzugshüllen bestehen in der Regel aus Polymerschläuchen, die meist aus Polyethylen oder Tetrafluorethylen hergestellt sind. Die Wandstärke dieser Polymerschläuche wird dabei so bemessen, dass sie dem Expansionsdruck des zusammengefalteten Gefäßimplantats Stand hält und auch über der Zeit stabil bleibt, sowie keinem thermischen Kriechen unterliegt. Dies bedeutet jedoch, dass die Rückzugshülle ein relativ hohes Flächenträgheitsmoment seines Querschnittprofils aufweist, und darüber hinaus sind Rückzugshüllen auch relativ steif in axialer Richtung, damit der Operateur nicht die Kontrolle über den Grad der Gefäßimplantatfreisetzung verliert. Aus diesen Gründen solle der Pusher auch steifer als die Rückzugshülle in axialer Richtung sein, um den oben beschriebenen Kräften hinreichend gut entgegenwirken zu können.

Die im Stand der Technik bekannten Einführsysteme umfassen daher in der Regel neben einem innenliegenden Drahtführungskatheter ein steifes Pusher-Katheterrohr sowie einen äußeren Hüllschlauch bzw. Hüllkatheter.

Es hat sich nun gezeigt, dass die im Stand der Technik bekannten Einführsysteme insbesondere bei Patienten mit stark gewundenen Gefäßen nur schwer oder gar nicht eingesetzt werden können, da es die Steifigkeit der Bestandteile, insbesondere des Pusher-Katheterrohrs nicht zulässt, das Einführsystem und mit diesem das Gefäßimplantat durch die gewundenen Gefäßverläufe vorzuschieben. Neben der Gefahr einer Gefäßwandperforation besteht darüber hinaus auch der Nachteil, dass mit den im Stand der Technik bekannten Einführsystemen der Stent bei stark gewundenen Gefäßen nicht mit hinreichender Präzision positioniert werden kann, dass ferner die Rückzugshülle Knicke bildet und dass durch diese Knickbildung der Kraftaufwand sogar erhöht wird, der zur Überwindung der Knickstellen in der Rückzugshülle angewandt werden müssen. Auch die Gefahr einer Knickbildung in Führungsdrahtkatheter gegenüber dem darauf geführten Pusher-Katheterrohr besteht, und zwar an der Übergangsstelle zum Pusher-Katheter, was ebenfalls den Abbruch des Einführvorganges notwendig macht.

Vor diesem Hintergrund liegt der vorliegenden Erfindung die Aufgabe zugrunde, eine Pusher-Baugruppe bzw. ein die Pusher-Baugruppe aufweisendes Einführsystem der eingangs genannten Art zu schaffen, bei dem die vorstehend genannten Nachteile vermieden werden. Insbesondere soll die neue Pusher-Baugruppe zu einer Erhöhung der Flexibilität des Einführsystems ermöglichen, um auch Patienten, deren Gefäße stark gewunden sind, behandeln zu können.

Gemäß der vorliegenden Erfindung wird diese Aufgabe und andere durch eine Pusher-Baugruppe gemäß den beiliegenden Ansprüchen gelöst, und unter anderem dadurch, dass bei der eingangs genannten Pusher-Baugruppe die Pusher-Anordnung durch einzelne gleichartige, segmentartige Elemente gebildet ist, die hintereinander und aneinander angrenzend sowie zur proximalen Angrenzung an das Gefäßimplantat auf dem Katheterschlauch angeordnet sind.

Die der Erfindung zugrundeliegende Aufgabe wird ferner durch ein Einführsystem gelöst, das die genannte erfindungsgemäße Pusher-Baugruppe aufweist.

Die der Erfindung zugrundeliegende Aufgabe wird auf diese Weise vollkommen gelöst.

Bei der erfindungsgemäßen Pusher-Baugruppe liegen erfindungsgemäß segmentartige Elemente vor, die auf dem Katheterschlauch sozusagen "perlenartig" aufgefädelt sind, und durch deren Aneinanderreihung auf dem Katheterschlauch eine Pusher-Anordnung gebildet wird, die aufgrund der Steifigkeit der einzelnen sementartigen Elemente die Längsstabilität der Pusher-Anordnung nicht beeinträchtigen, die jedoch aufgrund der Flexibilität einzelnen Elemente zueinander eine Gesamtflexibilität der Pusher-Anordnung in alle Richtungen ermöglicht. So kann die Pusher-Baugruppe, also die Kombination aus Katheterschlauch und darauf angeordneten segmentartigen Elementen, relativ einfach gebogen und damit insbesondere in gekrümmte Gefäße einfach eingeführt werden, wo sich die Baugruppe der Anatomie des Gefäßes anpassen kann. Dies wird dadurch erreicht, dass die Pusher-Anordnung, die aufgrund der einzelnen auf den Katheterschlauch aufgefädelten Elementen ja segmentiert ist, einfach und ohne Druck- oder Kraftbelastung eine Biegung in verschiedene Richtungen vollführen kann. Gleichzeitig bieten die segmentartigen Elemente ― bzw. "Segmente" durch deren kompakte Aneinanderreihung in Längsrichtung eine hinreichende Pusher-Kraft, die der Kraft, die durch das Zurückziehen der Rückzugshülle ausgeübt wird, wie bei einem einteiligen steifen Pusher-Katheterrohr entgegenwirken kann.

Während also die einzelnen segmentartigen Elemente der Pusher-Anordnung aus einem relativ steifen Material sein können, verleihen diese der Pusher-Baugruppe insgesamt dennoch eine hohe Flexibilität, da der Führungsgradkatheter in dem Bereich, in dem die einzelnen Elemente aufgefädelt sind, und an das Gefäßimplantat im geladenen Zustand anstößt, flexibel gebogen werden kann. Dies bedeutet eine vorteilhafte Verbesserung gegenüber Pusher-Kathetern, die wie im Stand der Technik aus einem steifen extrudierten Schlauch oder Rohr bestehen, und die nur schwer gebogen werden können, und in diesem Bereich wenig flexibel sind.

Dabei ist es bevorzugt, wenn die Elemente gleichartig, vorzugsweise identisch sind. Erfindungsgemäß weisen die Elemente eine Form auf, die ausgewählt ist aus, kugelförmig bzw. im Wesentlichen kugelförmig, oliven- bzw. eiförmig, kegelförmig, stumpfkegelförmig, glockenförmig, wobei die Elemente ferner ein proximales und ein distales Ende aufweisen und eine mittige Bohrung zur Bildung einer proximalen und einer distalen Öffnung und eines durch die die Elemente geführten Kanals, durch welchen hindurch der Drahtführungskatheterschlauch führbar ist.

Dabei wird unter "im Wesentlichen" verstanden, dass die Form einer Kugel oder einer Olive nicht streng eingehalten werden muss, dass aber die Grundform einer Kugel oder Olive oder eines Eis oder dergleichen erkennbar ist, und die Kugelform oder die Olivenform oder dergleichen aber bspw. gekappte Enden besitzen, eine ovalere Form mit abgekappten und/oder runden Enden, etc.

Durch die Bohrung weisen die einzelnen segmentartigen Elemente also eine distale und eine proximale Öffnung auf.

In diesem Zusammenhang versteht sich, dass bei der erfindungsgemäßen Pusher-Baugruppe die Form der segmentartigen Elemente derart ist, dass deren nach außen, d.h. zur Gefäßwand gerichteten Oberfläche zumindest teilweise gewölbt bzw. abgerundet ist, und dass sie weiter vorzugsweise mittig eine Bohrung aufweisen, durch welche hindurch der Katheterschlauch geführt ist. Die einzelnen Elemente sind also in der Tat wie Perlen auf eine Schnur aufgefädelt.

Dabei ist bevorzugt, wenn mindestens vier einzelne Elemente als Pusher-Anordnung vorliegen; in Ausführungsformen der Erfindung können auch weniger als vier oder aber genau vier, oder fünf, sechs, sieben, acht, neun, zehn, elf oder zwölf, einzelne Elemente vorliegen, wobei dem Fachmann klar sein wird, dass die Anzahl der einzelnen segmentartigen Elemente von deren jeweiliger Länge und Größe sowie von dem einzusetzenden Einführsystem bzw. der Arbeitslänge und letztendlich auch von dem zu behandelnden Gefäß abhängen wird, und dem Fachmann wird nach Durchlesen der vorliegenden Lehre klar sein, wie viele Element vorliegen müssen, um die erfindungsgemäße Pusher-Baugruppe zu verwirklichen.

Das Material, das für die segmentartigen Elemente gemäß der vorliegenden Erfindung vorzugsweise eingesetzt wird, ist dabei ausgewählt aus zumindest einem der folgenden: Perflour (Ethylen-Propylen) Kunststoff (FEP), Polytetrafluorethylen (PTFE), Polyoxymethylen(POM), High density Polyethylen (HDPE) (PE hoher Dichte) oder Mischungen davon. Die einzelnen Elemente können dabei einen Härtegrad von zwischen 60 Shore A und 100 Shore D aufweisen (die Shore-Härte ist eine im Stand der Technik bekannte Kennzahl für Elastomere und Polymere) , was zeigt, dass durch die Hintereinanderreihung dieser harten bzw. steifen Elemente hinsichtlich der Längsstabilität der Pusher-Anordnung keinerlei Abstriche gemacht werden, sondern im Gegenteil sogar eine höhere Längsstabilität erzielt werden kann.

Vorliegend und durchgehend durch die Beschreibung wird unter einem "Katheterschlauch" jeder schlauch- oder rohrförmige Katheter verstanden, der üblicherweise im Gebiet der Einführsysteme für hierauf zu ladende Gefäßimplantate eingesetzt wird, und im Allgemeinen werden hierunter Röhrchen oder Schläuche verschiedener Durchmesser und Materialien verstanden, die je nach Einsatz/Patient/Gefäß variieren können.

Gemäß einer weiteren Ausführungsform der vorliegenden Erfindung ist bevorzugt, wenn zumindest eines der Elemente, vorzugsweise alle Elemente eine Oberfläche aufweisen, die Rillen und/oder längliche Vertiefungen aufweist, wobei die Rillen und/oder länglichen Vertiefungen auf der Oberfläche im Wesentlichen parallel zum Katheterrschlauch und damit parallel zur Gefäßrichtung verlaufen.

Diese Merkmale haben den Vorteil, dass gegenüber der auch über die Pusher-Anordnung geführten Rückzugshülle nur eine punktförmige Belastung zu dieser hin bewirkt wird, wodurch wiederrum eine geringere Reibung beim Rückziehen der Rückzugshülle erreicht wird und als Konsequenz weniger Kraft beim Zurückziehen und Freisetzen durch den Operateur aufgewendet werden muss. Dadurch wird auch ein ruckartiges und/oder ungleichmäßiges Freisetzen vermieden, weshalb insgesamt eine präzisere und einfachere Gefäßimplantat-Freisetzung möglich ist.

Gemäß einer weiteren Ausführungsform der vorliegenden Erfindung können die einzelnen, segmentartigen Elemente ferner eine Beschichtung aufweisen, mit der die Reibung weiter verkleinert werden kann. Dabei ist die Beschichtung vorzugsweise ausgewählt aus zumindest einem der folgenden: hydrophile Beschichtung (Polyvinylalkohol), Silikonöle oder Parylene Beschichtung, oder Mischungen davon.

Die Rillen/Vertiefungen in der Oberfläche der Elemente ermöglichen außerdem eine gute Spülbarkeit der Pusher-Anordnung, wodurch verhindert wird, dass nach dem Spülen Luftbläschen an der Oberfläche haften bleiben oder dort entstehen. Dies wiederum ermöglicht ein vereinfachtes Spülen der Einführvorrichtung, was einen essentiellen Schritt vor der Einführung des Einführsystems in ein Gefäß darstellt, da das Einführen von Luft in die Gefäße unbedingt vermieden und verhindert werden muss.

Gemäß einer weiteren Ausführungsform sind die Elemente dabei nicht direkt und fest miteinander verbunden oder verbindbar, sondern stoßen lediglich aneinander an, bzw. liegen lediglich aneinander angrenzend auf dem Drahtführungskatheterschlauch vor. Dabei wird bei der Anbringung der Elemente auf dem Drahtführungskatheterschlauch darauf geachtet, dass die Elemente möglichst kompakt und ohne Zwischenräume zueinander "aufgefädelt" werden, um ein Verrutschen der Pusher-Anordnung bzw. der Elemente untereinander zu vermeiden.

Vorliegend und durchgehend durch die Erfindung wird unter dem Begriff "distal" derjenige Teil oder Abschnitt der Pusher-Baugruppe und/oder des Einführsystems insgesamt verstanden, der weiter vom Operateur entfernt liegt, und der Abschnitt/Teil, der näher bei der handhabenden Person liegt, als "proximal". Entsprechend ist die "distale" Richtung diejenige Richtung, die vom Operateur weg weist, und die "proximale" Richtung, die zum Operateur hinweist.

Gemäß einer weiteren Ausführungsform kann vorgesehen sein, dass die Elemente teilweise ineinander eingreifen. Gemäß einer weiteren bevorzugten Ausführungsform sind die Formen der segmentartigen Elemente also derart beschaffen, dass im Bereich der durch die Bohrung vorgesehenen proximalen Öffnung eines ersten segmentartigen Elements eine trichterförmige, auf die proximale Öffnung nach innen zulaufende Einpassung vorgesehen ist, in welche das distale Ende bzw. die distale Öffnung eines proximal voran angeordneten segmentartigen Elements passend bzw. mit etwas Spielraum eingreifen kann.

Auch mit dieser Ausführungsform ist damit gewährleistet, dass die Pusher-Anordnung aus den einzelnen Elementen hinreichend flexibel ist, da der Katheter, auf dem die Elemente getragen werden, bzw. aufgefädelt sind, nach wie vor mit diesen flexibel gebogen werden kann.

Gemäß einer weiteren Ausführungsform kann vorgesehen sein, dass die einzelnen Elemente durch einen Druckverschluss miteinander verbindbar sind. Vorzugsweise wird dabei eine unlösbare Ringschnappverbindung vorgesehen.

Gemäß einer noch weiteren Ausführungsform der erfindungsgemäßen Pusher-Baugruppe ist zumindest das proximal und/oder distal äußerste Element jeweils am Katheterschlauch fest fixiert.

Bei dieser Ausführungsform ist somit bevorzugt vorgesehen, dass mindestens das auf dem Katheterschlauch am weitesten proximal und/oder das am weitesten distal gelegene Element fest mit dem hierdurch geführten Katheterschlauch verbunden, vorzugsweise verklebt, ist. Die einzelnen Elemente jedoch sind untereinander nicht verklebt, und liegen daher gemäß einer bevorzugten Ausführungsform auch theoretisch verschiebbar auf dem Katheterschlauch vor, werden jedoch durch die kompakte Auffädelung und Fixierung der äußersten Elemente bzw. des äußersten Elements unverschiebbar bzw. unverrückbar auf dem Katheterschlauch platziert.

Die hierin offenbarte und weiter oben beschriebene Pusher-Baugruppe kann ferner erfindungsgemäß zur Verwendung in einem Einführsystem für ein Gefäßimplantat in ein Gefäß eines Patienten eingesetzt werden.

Bei dem Gefäß des Patienten kann es sich um ein Blutgefäß oder aber um ein anderes Körperlumen handeln, wie beispielsweise den Gallengang. Dem Fachmann wird dabei klar sein, dass die Pusher-Baugruppe und das erfindungsgemäße Einführsystem mit der erfindungsgemäßen Pusher-Baugruppe bei den unterschiedlichsten Gefäßimplantaten, insbesondere Stents oder Stentgrafts, eingesetzt werden kann, wobei ein Einsatz bei großen Durchmessern der Blutgefäße besonders vorteilhaft ist.

Dementsprechend betrifft die vorliegende Erfindung auch ein Einführsystem zum Einbringen eines Gefäßimplantats in ein Gefäß eines Patienten, das neben der erfindungsgemäßen Pusher-Baugruppe auch mindestens eines, vorzugsweise mehrere, und noch bevorzugter sämtliche der folgenden Komponenten aufweist, nämlich ein Führungsdraht, eine Rückzugshülle zum Komprimieren des Gefäßimplantats in dessen geladenem Zustand, sowie ein Gefäßimplantat, sowie eine proximale Handhabungsanordnung, mit der die Einführung und Freisetzung des Gefäßimplantats proximal betätigt werden kann .

Weitere Vorteile und Merkmale ergeben sich aus der nachfolgenden Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ein Ausführungsbeispiel der Erfindung ist in den beiliegenden Figuren dargestellt und wird in Bezug auf diese nachstehend näher beschrieben. Es zeigen:
- Fig. 1: eine schematische Darstellung eines einzelnen segmentartigen Elements der Pusher-Baugruppe in perspektivischer, seitlicher Ansicht auf die proximale Öffnung (1A); in perspektivischer seitlicher Ansicht auf die distale Öffnung (1B); sowie in direkter seitlicher Draufsicht (Fig. 1C);
- Fig. 2: eine schematische Darstellung einer beispielhaften Ausführungsform der erfindungsgemäßen Pusher-Baugruppe in seitlicher, schräger Draufsicht(Fig. 2A) sowie in einer weiteren seitlichen Perspektive (Fig. 2B); und
- Fig. 3: eine schematische und ausschnittsweise Darstellung der Pusher-Baugruppe mit distal nachgelagertem Gefäßimplantat mit darüber angeordneter Rückzugshülle, im noch geladenen Zustand (Fig. 3A); sowie im teilweise freigesetzten Zustand (Fig. 3B).

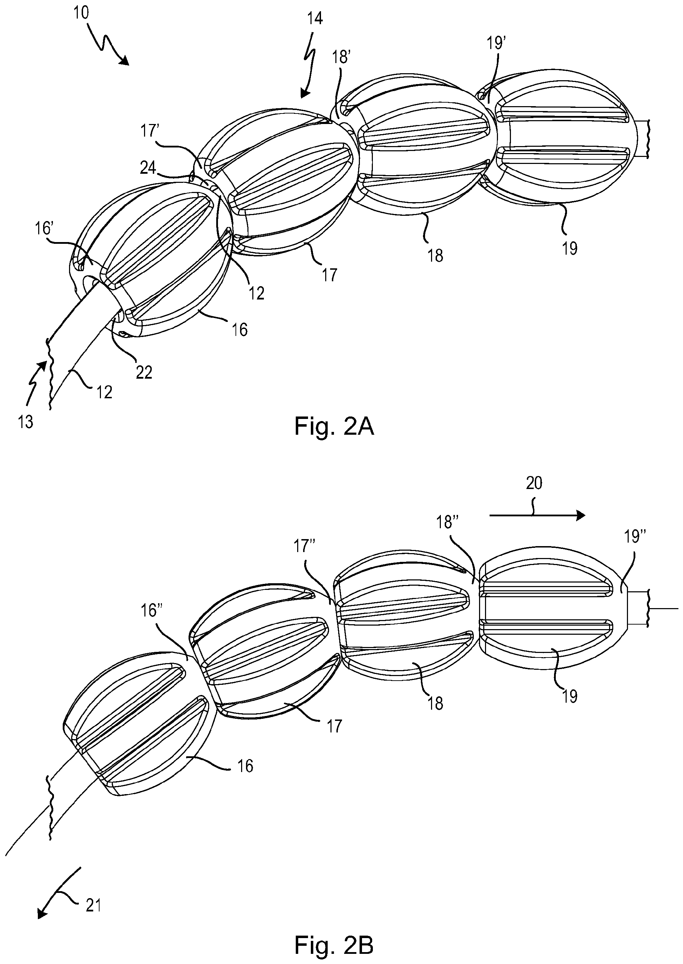

In Fig. 2A ist mit 10 insgesamt eine Pusher-Baugruppe bezeichnet, die einen Katheterschlauch 12 aufweist, welcher ein Lumen 13 zur Aufnahme eines Führungsdrahtes (nicht gezeigt) umfasst, sowie eine Pusher-Anordnung 14, die, wie in Fig. 2A und 2B gezeigt, aus vier einzelnen, segmentartigen Elementen 16, 17, 18, 19 gebildet ist. Es versteht sich, dass die Pusher-Anordnung 14 auch mehr Elemente aufweisen kann, und nicht auf die in den Figuren gezeigte Anzahl beschränkt ist.

Eine ähnliche Ansicht findet sich in Fig. 2B, wobei letztere Ansicht eine volle Seitenansicht ist.

Wie Fig. 2A zu entnehmen ist, ist die Form der Elemente 16, 17, 18, 19 kugel- bzw. eiförmig, bzw. im Querschnitt oval, und die Elemente 16, 17, 18, 19 weisen dadurch jeweils ein proximales Ende 16', 17', 18' und 19' auf, sowie jeweils ein distales Ende 16", 17", 18" und 19". Die Enden 16', 16", 17' 17", 18', 18", 19' und 19" sind dabei "stumpf" bzw. erscheinen "abgeschnitten" oder "gekappt".

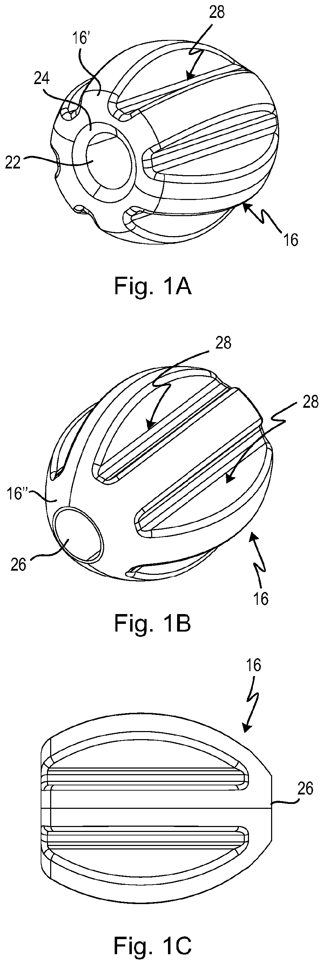

Die Elemente 16, 17, 18 und 19 weisen jeweils durchgehende mittige Bohrungen auf, weshalb sich an den proximalen Enden 16', 17', 18' und 19' jeweils eine proximale Öffnung befindet, die in Fig. 1A exemplarisch anhand des Elements 16 mit 22 gezeigt ist. Ferner weisen die Elemente 16, 17, 18 und 19 an ihrem distalen Ende 16", 17", 18" und 19" jeweils eine distale Öffnung 26 auf, die in der Darstellung des Elements 16 in Fig. 1 gut sichtbar ist. Über die Bohrungen und Öffnungen 22, 26, und der sich daran anschließenden Öffnungen und Bohrungen der Elemente 17, 18, und 19 wird der Katheterschlauch 12 durch die Elemente 16, 17, 18 und 19 hindurchgeführt und die einzelnen Elemente 16, 17, 18, 19 dadurch auf dem Katheterschlauch 12 aufgefädelt.

Der Pfeil 20 in Fig. 2B gibt dabei die distale Richtung an, mithin also diejenige Richtung, die vom Anwender oder Operateur weg führt, und der Pfeil 21 die proximale Richtung, mithin also die Richtung, die in Richtung des Operateurs führt.

Bezugnehmend auf Fig. 1, ist dort das Element 16 in Einzeldarstellung beispielhaft für die Elemente 16, 17, 18 und 19 gezeigt, die identisch aufgebaut sind. Es versteht sich, dass aber bspw. das jeweils am äußersten distal und/oder proximal vorgesehene Element, anders als die anderen segmentartigen Elemente ausgebildet sein kann/können Fig. 1A zeigt eine schräge perspektivische Ansicht auf die proximale Öffnung 22 des Elements 16, die an dessen proximalen Ende 16' vorgesehen ist. Fig. 1B zeigt eine schräge perspektivische Ansicht auf die distale Öffnung 26 am distalen Ende 16".Fig. 1C schließlich zeigt eine Seitendraufsicht auf das Element 16, das im Übrigen in seinem Aufbau und in seiner Größe identisch ist mit den anderen Elementen 17, 18 und 19.

Fig. 1A ist zu entnehmen, dass am proximalen Ende 16' um die proximale Öffnung 22 eine trichterartig nach innen und auf die Öffnung zulaufende Einsparung 24 gebildet ist. Fig. 1B, das die distale Öffnung 26 des Elements 16 an dessen distalen Ende 16" zeigt, ist zu entnehmen, dass dieses Ende 16" keine solche trichterförmig auf die Öffnung 26 zulaufende Einsparung oder Einfräsung oder Rand aufweist. Die distale Öffnung 26, die sich in der gleichen Form auch an den Elementen 17, 18 und 19 befindet, bzw. das distale Ende 16" ist passgenau auf das jeweils sich in distale Richtung anschließende proximale Ende 17' des nächsten Elements 17 angepasst und greift dadurch teilweise bzw. geringfügig in die trichterförmige Einsparung 24 am proximalen Ende 17' des nächsten Elements 17 ein, wodurch praktisch ein durch beide Elemente 16, 17 durchgehender Kanal für den Katheterschlauch 12 gebildet wird. Gleichzeitig erhalten die sich über die Öffnungen 22 und 26 anschließenden Elemente, insbesondere auch durch die trichterförmige Einsparung einen gewissen Bewegungs-Spielraum, der die Flexibilität der Pusher-Anordnung 14 begründet. Die weiteren Elemente 18 und 19 werden in gleicher Weise aneinandergereiht, bzw. auf den Katheterschlauch 12 "aufgefädelt".

Den Figuren 1 bis 3 ist ferner zu entnehmen, dass die Elemente 16, 17, 18 und 19 Rillen bzw. längliche Vertiefungen 28 in deren nach außen und zur Gefäßwand gerichteten Oberfläche besitzt, die wie Kerben in die Form der Elemente 16, 17, 18 und 19 eingefügt sind. Die Rillen 28 sind in dem in den Figuren dargestellten Beispiel umlaufend auf der Oberfläche der Elemente 16, 17, 18 und 19 regelmäßig beabstandet, wobei in dem in den Figuren gezeigten Beispiel für die Elemente 16, 17, 18 und 19 jeweils sechs Rillen 28 vorgesehen sind, durch welche sich regelmäßig beabstandete Vertiefungen und Erhebungen bilden.

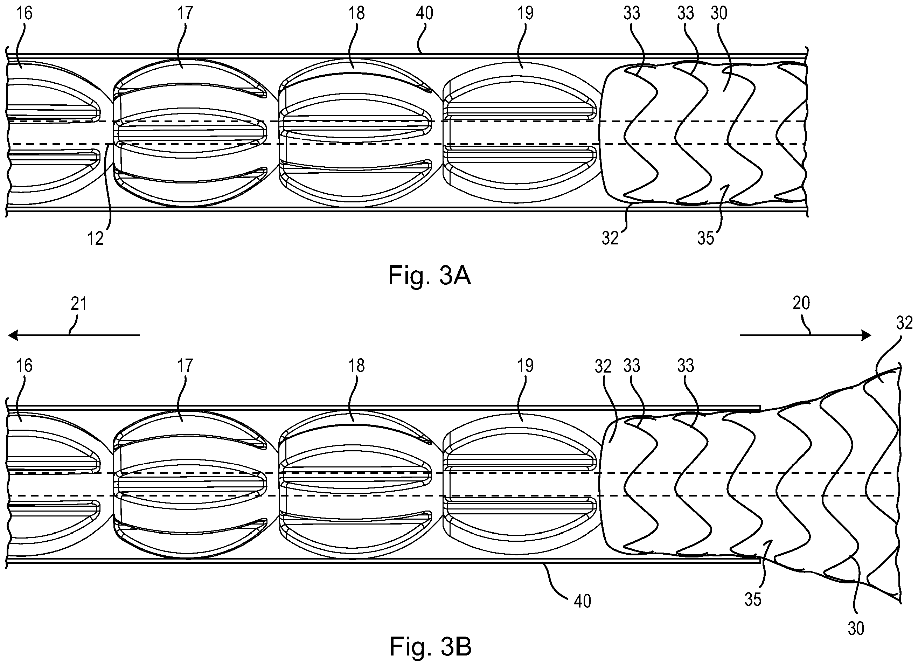

Durch diese Rillen 28 der nach außen weisenden Oberfläche der Elemente 16, 17, 18, und 19 ist die Gesamtanlagefläche, mit der die Pusher-Anordnung 14 mit einer Rückzugshülle (siehe Bezugszeichne 40 in Fig. 3A und 3B) in Berührung kommt deutlich verringert im Vergleich zu einer Oberfläche einer Pusher-Anordnung, die keine Rillen oder Vertiefungen 28 und keine einzelnen abgerundeten Elemente aufweist.

Die Rillen bzw. Vertiefungen 28 sind dabei parallel zum durch das Element hindurchgeführten Katheterschlauch und vom proximalen Ende 22 bis zum distalen Ende 22 geführt, und damit auch parallel zum Gefäß und ggf. Blutfluss.

Die Aneinanderreihung der Elemente 16, 17, 18 und 19 ist in den Fig. 2A und 2B dargestellt: Zu erkennen ist, dass die einzelnen Elemente 16, 17, 18 und 19 derart direkt hintereinander angeordnet sind, dass das distale Ende 16" eines ersten Elements 16 in die trichterförmig auf die proximale Öffnung 22 zulaufende Einbuchtung bzw. Einsparung 24 des proximalen Endes 17' des zweiten Elements 17 teilweise eingebracht ist bzw. passend eingreift. Dadurch stoßen die Elemente 16 und 17 direkt aneinander an.

Fig. 3 zeigt schließlich die in Fig. 1 und 2 dargestellte Pusher-Baugruppe mit einem auf einem distalen Abschnitt des Katheterschlauchs 12 (in Fig. 3A und 3B der Übersichtlichkeit halber gestrichelt dargestellt) geladenen Gefäßimplantat 30, dessen distales Ende 32 direkt an das distale Ende des Elements 19 anliegt. Dabei handelt es sich bei dem in Fig. 3 beispielhaft dargestellten Gefäßimplantat 30 um einen Stentgraft mit hintereinander angeordneten Stentringen 33 aus selbstexpandierendem Material mit einem diese verbindenden Implantatmaterial 35.

Eine Rückzugshülle 40 hält in Fig. 3A das Gefäßimplantat 30 im komprimierten Zustand und umgibt dabei auch die einzelnen Elemente 16, 17, 18, 19, die die Pusher-Anordnung 14 darstellen, sowie den mittig durch die Elemente 16, 17, 18, 19 bzw. durch deren Bohrungen und Öffnungen 22, 26, geführten Katheterschlauch 12. Die Rückzugshülle 40 ist bis zum proximalen Handhabungsende (nicht dargestellt) des Einführsystems geführt bzw. mit diesem verbunden, und kann vom proximalen Handhabungsende des Einführsystems betätigt werden.

In Fig. 3B ist ein erster Freisetzungsschritt des Gefäßimplantats 30 dargestellt: Hier wurde im Vergleich zu Fig. 3A die Rückzugshülle 40 zum Freisetzen des Gefäßimplantats 30 bereits ein Stück in proximale Richtung 21 gezogen. Beim Zurückziehen der Rückzugshülle 40 muss der Operateur eine bestimmte Kraft anwenden, da die Rückzugshülle aufgrund derer komprimierenden Wirkung auf das Gefäßimplantat 30 relativ große Kräfte ausübt, und da zusätzlich beim Zurückziehen Reibungskräfte zwischen der Rückzugshülle 40 und dem Gefäßimplantat sowie der Pusher-Baugruppe auftreten.

Fig. 3B ist nun zu entnehmen, dass nach einem ersten Schritt des Zurückziehens der Rückzugshülle 40 in die proximale Richtung 22 das Gefäßimplantat an seinem distale Richtung weisenden Ende 34 dort, wo es nicht mehr von der Rückzugshülle komprimiert gehalten wird, radial expandiert ist und sich an die Gefäßwand (nicht gezeigt) angelegen und dort verankern kann. Die Pusher-Anordnung 14, gebildet aus den Elementen 16, 17, 18 und 19, hält dabei, also beim Zurückziehen der Rückzugshülle 40, aufgrund ihrer guten Längsstabilität den Kräften, die beim Zurückziehen der Rückzugshülle 40 auftreten, entgegen und hält dadurch das Gefäßimplantat 30 an der korrekten Position im Gefäß.

Nachdem das Gefäßimplantat 30 vollständig freigesetzt ist, kann die Pusher-Anordnung 14 bzw. die Pusher-Baugruppe 10, bzw. vielmehr das Einführsystem als solches aus dem Gefäß entfernt werden.

## Patentansprüche

1. Pusher-Baugruppe (10) für ein Einführsystem für ein Gefäßimplantat (30), wobei die Pusher-Baugruppe (10) einen Katheterschlauch (12) aufweist, welcher ein Lumen (13) zur Aufnahme eines Führungsdrahtes aufweist, sowie einen proximalen und einen sich daran anschließenden distalen Katheterschlauchabschnitt, wobei der distale Katheterschlauchabschnitt zumindest teilweise zur verschiebbaren Aufnahme eines Gefäßimplantats (30) hierauf vorgesehen ist, und wobei der Katheterschlauch (12) ferner proximal angrenzend zu einem auf den Katheterschlauch (12) geladenen Gefäßimplantat (30) eine Pusher-Anordnung (14) zum Freisetzen des Gefäßimplantats (30) aufweist, **dadurch gekennzeichnet, dass** die Pusher-Anordnung (30) durch einzelne, segmentartige Elemente (16, 17, 18, 19) gebildet ist, die hintereinander und aneinander angrenzend sowie zur proximalen Angrenzung an das Gefäßimplantat (30) auf dem Katheterschlauch (12) angeordnet sind, wobei die einzelnen segmentartigen Elemente (16, 17, 18, 19) eine Form aufweisen, die ausgewählt ist aus, kugelförmig bzw. im Wesentlichen kugelförmig, eiförmig, kegelförmig, stumpfkegelförmig, glockenförmig, wobei die Elemente (16, 17, 18, 19) ferner ein proximales (16', 17', 18', 19') und ein distales (16", 17", 18", 19") Ende aufweisen und eine mittige Bohrung zur Bildung einer proximalen (22) und einer distalen (26) Öffnung und eines durch die die Elemente (16, 17, 18, 19) geführten Kanals, durch welchen hindurch der Katheterschlauch (12) führbar ist.

2. Pusher-Baugruppe (10) nach Anspruch 1, **dadurch gekennzeichnet, dass** die Elemente (16, 17, 18, 19) zumindest gleichartig, vorzugswiese identisch, sind.

3. Pusher-Baugruppe (10) nach einem der Ansprüche 1 oder 2, **dadurch gekennzeichnet, dass** mindestens vier Elemente (16, 17, 18, 19) vorgesehen sind.

4. Pusher-Baugruppe (10) nach einem der Ansprüche 1 bis 3, **dadurch gekennzeichnet, dass** zumindest eines der Elemente (16, 17, 18, 19) auf seiner der Gefäßwand zugewandten Oberfläche Rillen (28) aufweist, wobei die Rillen (28) auf der Oberfläche im Wesentlichen parallel zum Katheterschlauch (12) und von einem proximalen Ende 22 bis zu einem distalen Ende (26) der Elemente (16, 17, 18, 19) verlaufen.

5. Pusher-Baugruppe (10) nach einem der Ansprüche 1 bis 4, **dadurch gekennzeichnet, dass** die Elemente (16, 17, 18, 19) nicht direkt und fest miteinander verbunden sind oder verbindbar sind, sondern lediglich aneinander angrenzend vorliegen.

6. Pusher-Baugruppe (10) nach einem der Ansprüche 1 bis 5, **dadurch gekennzeichnet, dass** die Elemente (16, 17, 18, 19) teilweise ineinander eingreifen.

7. Pusher-Baugruppe (10) nach einem der Ansprüche 2 bis 6, **dadurch gekennzeichnet, dass** am proximalen Ende (16', 17', 18', 19') eines Elements (16, 17, 18, 19) eine trichterförmige, auf die proximale Öffnung (22) nach innen zulaufende Einpassung (24) vorgesehen ist.

8. Pusher-Baugruppe (10) nach einem der Ansprüche 1 bis 7, **dadurch gekennzeichnet, dass** die einzelnen Elemente (16, 17, 18, 19) durch Druckverschluss miteinander verbindbar sind.

9. Pusher-Baugruppe (10) nach einem der Ansprüche 1 bis 8, **dadurch gekennzeichnet, dass** zumindest das proximal und/oder distal äußerste Element (16, 19) jeweils am Katheterschlauch (12) fest fixiert ist.

10. Pusher-Baugruppe (10) nach einem der Ansprüche 1 bis 9 zur Verwendung in einem Einführsystem für ein Gefäßimplantat (30) in ein Gefäß eines Patienten.

11. Einführsystem zum Einbringen eines Gefäßimplantats (30) in ein Gefäß eines Patienten, **dadurch gekennzeichnet, dass** es eine Pusher-Baugruppe (10) nach einem der Ansprüche 1 bis 10 aufweist.

12. Einführsystem nach Anspruch 11, **dadurch gekennzeichnet, dass** es ferner zumindest eines der folgenden aufweist, nämlich einen Führungsdraht, eine Rückzugshülle (40) zum Komprimieren des Gefäßimplantats (30) in dessen geladenen Zustand, ein Gefäßimplantat (30).

## Claims

1. A pusher assembly (10) for an insertion system for a vascular implant (30), wherein the pusher assembly (10) has a catheter tube (12) which has a lumen (13) for accommodating a guide wire and which has a proximal catheter tube portion and, adjoining the latter, a distal catheter tube portion, wherein the distal catheter tube portion is provided at least partially for movably accommodating a vascular implant (30) thereon, and wherein the catheter tube (12) moreover has a pusher unit (14) for releasing the vascular implant (30), said pusher unit (14) being proximally adjacent to a vascular implant (30) loaded onto the catheter tube (12), **characterized in that** the pusher unit (30) is formed by individual, segment-like elements (16, 17, 18, 19), which are arranged one behind another and adjacent to one another on the catheter tube (12) and proximally adjacent to the vascular implant (30), wherein the individual segment-like elements (16, 17, 18, 19) have a shape that is chosen from among spherical, or substantially spherical, oval, conical, frustoconical and bell-shaped, wherein the elements (16, 17, 18, 19) moreover have a proximal end (16', 17', 18', 19') and a distal end (16", 17", 18", 19") and a central bore for forming a proximal opening (22) and a distal opening (26) and for forming a channel which is routed through the elements (16, 17, 18, 19) and through which the catheter tube (12) can be guided.

2. The pusher assembly (10) as claimed in claim, **characterized in that** the elements (16, 17, 18, 19) are at least similar, preferably identical.

3. The pusher assembly (10) as claimed in one of claims 1 or 2, **characterized in that** at least four elements (16, 17, 18, 19) are provided.

4. The pusher assembly (10) as claimed in one of claims 1 through 3, **characterized in that** at least one of the elements (16, 17, 18, 19) has grooves (28) on its surface directed toward the vessel wall, wherein the grooves (28) on the surface extend substantially parallel to the catheter tube (12) and from a proximal end 22 to a distal end (26) of the elements (16, 17, 18, 19).

5. The pusher assembly (10) as claimed in one of claims 1 through 4, **characterized in that** the elements (16, 17, 18, 19) are not connected or connectable to one another directly and rigidly but instead merely lie adjacent to one another.

6. The pusher assembly (10) as claimed in one of claims 1 through 5, **characterized in that** the elements (16, 17, 18, 19) partially engage in one another.

7. The pusher assembly (10) as claimed in one of claims 2 through 6, **characterized in that** a funnel-shaped recess (24) extending inward to the proximal opening (22) is provided on the proximal end (16', 17', 18', 19') of an element (16, 17, 18, 19).

8. The pusher assembly (10) as claimed in one of claims 1 through 7, **characterized in that** the individual elements (16, 17, 18, 19) are connectable to one another by compressive closure.

9. The pusher assembly (10) as claimed in one of claims 1 through 8, **characterized in that** at least the outermost proximal and/or distal element (16, 19) is fixed rigidly on the catheter tube (12).

10. The pusher assembly (10) as claimed in one of claims 1 through 9, for use in an insertion system for inserting a vascular implant (30) into a vessel of a patient.

11. An insertion system for introducing a vascular implant (30) into a vessel of a patient, **characterized in that** it has a pusher assembly (10) as claimed in one of claims 1 through 10.

12. The insertion system as claimed in claim 11, **characterized in that** it moreover has one of the following: a guide wire, a withdrawal sleeve (40) for compressing the vascular implant (30) in the loaded state thereof, a vascular implant (30).

## Revendications

1. Ensemble poussoir (10) pour un système d'introduction pour un implant vasculaire (30), l'ensemble poussoir (10) comprenant un tuyau de cathéter (12), qui comprend une lumière (13) pour la réception d'un fil-guide, ainsi qu'une section de tuyau de cathéter proximale et une section de tuyau de cathéter distale y faisant suite, la section de tuyau de cathéter distale étant prévue au moins en partie pour la réception en coulissement d'un implant vasculaire (30) sur celle-ci, et le tuyau de cathéter (12) comprenant en outre, de manière proximalement adjacente à un implant vasculaire (30) chargé sur le tuyau de cathéter (12), un agencement poussoir (14) pour la libération de l'implant vasculaire (30), **caractérisé en ce que** l'agencement poussoir (30) est formé par des éléments individuels (16, 17, 18, 19) de type segment, qui sont agencés les uns derrière les autres et adjacents les uns aux autres, ainsi que pour être proximalement adjacents à l'implant vasculaire (30) sur le tuyau de cathéter (12), les éléments individuels (16, 17, 18, 19) de type segment présentant une forme qui est choisie parmi une forme sphérique ou sensiblement sphérique, ovoïde, conique, tronconique, en forme de cloche, les éléments (16, 17, 18, 19) présentant en outre une extrémité proximale (16', 17', 18', 19') et une extrémité distale (16", 17", 18", 19"), et un alésage central pour la formation d'une ouverture proximale (22) et d'une ouverture distale (26) et d'un canal passant à travers les éléments (16, 17, 18, 19), à travers lequel le tuyau de cathéter (12) peut passer.

2. Ensemble poussoir (10) selon la revendication 1, **caractérisé en ce que** les éléments (16, 17, 18, 19) sont au moins similaires, de préférence identiques.

3. Ensemble poussoir (10) selon l'une quelconque des revendications 1 ou 2, **caractérisé en ce qu'**au moins quatre éléments (16, 17, 18, 19) sont prévus.

4. Ensemble poussoir (10) selon l'une quelconque des revendications 1 à 3, **caractérisé en ce qu'**au moins un des éléments (16, 17, 18, 19) présente des rainures (28) sur sa surface tournée vers la paroi vasculaire, les rainures (28) sur la surface s'étendant essentiellement parallèlement au tuyau de cathéter (12) et d'une extrémité proximale (22) à une extrémité distale (26) des éléments (16, 17, 18, 19).

5. Ensemble poussoir (10) selon l'une quelconque des revendications 1 à 4, **caractérisé en ce que** les éléments (16, 17, 18, 19) ne sont pas reliés ou aptes à être reliés directement et fermement les uns aux autres, mais sont simplement adjacents les uns aux autres.

6. Ensemble poussoir (10) selon l'une quelconque des revendications 1 à 5, **caractérisé en ce que** les éléments (16, 17, 18, 19) pénètrent partiellement les uns dans les autres.

7. Ensemble poussoir (10) selon l'une quelconque des revendications 2 à 6, **caractérisé en ce qu'**un raccord en forme d'entonnoir (24) qui se rétrécit vers l'intérieur en direction de l'ouverture proximale (22) est prévu à l'extrémité proximale (16', 17', 18', 19') d'un élément (16, 17, 18, 19).

8. Ensemble poussoir (10) selon l'une quelconque des revendications 1 à 7, **caractérisé en ce que** les éléments individuels (16, 17, 18, 19) sont aptes à être reliés les uns aux autres par une fermeture à pression.

9. Ensemble poussoir (10) selon l'une quelconque des revendications 1 à 8, **caractérisé en ce qu'**au moins l'élément proximalement et/ou distalement le plus extérieur (16, 19) est à chaque fois fermement fixé au tuyau de cathéter (12).

10. Ensemble poussoir (10) selon l'une quelconque des revendications 1 à 9, destiné à être utilisé dans un système d'introduction pour un implant vasculaire (30) dans un vaisseau d'un patient.

11. Système d'introduction pour l'insertion d'un implant vasculaire (30) dans un vaisseau d'un patient, **caractérisé en ce qu'**il comprend un ensemble poussoir (10) selon l'une quelconque des revendications 1 à 10.

12. Système d'introduction selon la revendication 11, **caractérisé en ce qu'**il comprend en outre au moins un des éléments suivants, à savoir un fil-guide, une gaine de rétraction (40) pour la compression de l'implant vasculaire (30) dans son état chargé, un implant vasculaire (30).
